# EUROPÄISCHE PATENTSCHRIFT

(11) **EP 1 356 287 B1**
(45) Veröffentlichungstag und Bekanntmachung des Hinweises auf die Patenterteilung: **10.09.2008**
(21) Anmeldenummer: 01991820.0
(22) Anmeldetag: 11.12.2001
(51) Int. Cl.: G01N 33/50

(54) **REPLIKATIONSASSAY ZUR AUFFINDUNG ANTIVIRALER SUBSTANZEN**
REPLICATION ASSAY FOR DETECTING ANTI-VIRAL SUBSTANCES
TEST DE REPLICATION POUR LA RECHERCHE DE SUBSTANCES ANTIVIRALES

(30) Priorität: 18.12.2000 DE 10063111
(43) Veröffentlichungstag der Anmeldung: 29.10.2003
(73) Patentinhaber: AiCuris GmbH & Co. KG, 42117 Wuppertal (DE)
(72) Erfinder: PAESSENS, Arnold, 42781 Haan (DE); DITTMER, Frank, 40627 Düsseldorf (DE)
(74) Vertreter: Weller, Wolfgang
(86) Internationale Anmeldenummer: PCT/EP2001/014516
(87) Internationale Veröffentlichungsnummer: WO 2002/050536

(56) Entgegenhaltungen:
- WO-A-00/54839
- WO-A-90/01870
- US-A- 5 055 457
- US-A- 5 073 571
- GHEZZI SILVIA ET AL: "Inhibition of CXCR4-dependent HIV-1 infection by extracellular HIV-1 Tat." BIOCHEMICAL AND BIOPHYSICAL RESEARCH COMMUNICATIONS, Bd. 270, Nr. 3, 21. April 2000 (2000-04-21), Seiten 992-996, XP002224947 ISSN: 0006-291X

## Beschreibung

Die vorliegende Erfindung betrifft ein Replikationsassay zum Auffinden antiviral wirksamer Hemmstoffe, bestehend aus der Kombination von einem natürlichem Virus-Isolat, suspendierten Zielzellen und Zellkulturmedium enthaltend foetales worin die Konzentration des foetalen Kälberserum weniger als 5 Vol % beträgt.

Die Suche nach neuen, besser wirkenden und besser verträglichen Wirkstoffen, die als Therapeutika zur Behandlung bei viralen Infektionen, insbesondere der Infektion mit HIV verwendet werden können, ist eine drängende Aufgabe der pharmazeutischen Forschung, im Rahmen derer hunderttausende Testverbindungen auf ihre Wirksamkeit hin untersucht werden müssen.

Dazu wird üblicherweise im enzymatischen Test die Wirkung auf virusspezifische Transkriptasen oder Proteasen, oder im in vitro Modell, die Wirkung der Testsubstanz auf das native, unveränderte Virus und dessen Verhalten an geeigneten Zielzellen untersucht.

Die große Zahl der zu testenden Proben bedingt erhebliche logistische Probleme, die nur durch Automatisierung der Testabläufe zu lösen sind.

Dies ist im Falle der enzymatischen Tests der Stand der Technik, wo große Probenmengen mit sogenannten High-Throughput - Screening Verfahren (HTS-Verfahren) untersucht werden können. Solche Tests sind auch im Falle der Enzyme von HIV durchgeführt worden. So wurde kürzlich mittels HTS ein Test an dem viruseigenen Enzym Integrase durchgeführt. (Yves Pommier et al. Antiviral Research 47 (2000) 139-148; Hazuda et al. J. Virol. 71, 7005-7011; Hazuda et al. Antiviral Chem. Chemother. 10,63-70). U.S-A-5055457 beschreibt ein Testsystem zum auffinden antiviral wirksamer teststoffe enthaltend einen replikations assay, in dem die konzentration von pötalem kälberserum 10% beträgt. Tests auf Basis von Proteinen und Enzymen eignen sich besonders gut für die Durchführung im HTS, weil für die verfügbaren Rcad-outs exakte und schnelle Auswertemöglichkeiten, beispielsweise als optische Verfahren durch Messung der Fluoreszenz, gegeben sind. Ein gutes Signal-Rauschverhältnis lässt sich durch Auswahl geeigneter Farbindikatoren erzielen. Allerdings ist der enzymatische Test prinzipiell mit dem Nachteil behaftet, dass er nur einen kleinen Teil aller am Replikationszyklus beteiligten viralen und oder zellulären Systeme erfasst.

Demgegenüber erlaubt die Untersuchung der Replikation von Viren in der Zellkultur die simultane Überprüfung aller an der Virusvermehrung beteiligten Schritte unter weitestgehend natürlichen Bedingungen. (Replikationsassay) .

Replikationsassays sind deshalb besonders geeignet, um unbekannte, viruskodierte und/oder wirtseigene Prozesse mit zu erfassen, die im enzymatischen Test prinzipiell nicht nachstellbar sind.

Betreffend HIV sind dies zum Beispiel das Attachment an und das Eindringen in die zu infizierende Zelle sowie die Wirkung virusspezifischer regulativer Mechanismen oder Prozesse, bei denen virus- und zelleigene Faktoren zusammenwirken.

Solche viruseigenen Schritte, die in vitro - wenn überhaupt - nur unter Zusatz außerordentlich komplexer Reaktionsadditive verlaufen, sind im Replikationsassay in geeigneten Wirtszellen abgebildet und funktionieren innerhalb des Rcplikationsprozesses des HIV optimal.

Darüber hinaus liefert der Replikationsassay zugleich Hinweise zur Zytotoxizität (Selektivitätsindex) der Testverbindungen und damit ein erstes Maß für die Einschätzung des Potentials der Wirkverbindung.

Der Replikationstest eignet sich vor diesem Hintergrund besonders, neue, wirksamere und besser verträgliche Hemmstoffe des HIV aufzufinden.

Allerdings ist der Replikationsassay mit dem prinzipiellen Nachteil belegt, dass der Endpunkt, nämlich die Beurteilung des Verhaltens der dem Virus ausgesetzten Zellen, bisher noch nicht im HTS-Verfahren bestimmt werden kann. Zwar ist es bekannt, gentechnisch veränderte und mit geeigneten Indikatorsystemen wie beispielsweise dem Luciferase-Gen versehene HIV-Varianten einzusetzen, doch verhalten sich solche durch das Einklonieren von Reporter-Genen veränderte Varianten des HIV nicht notwendig so wie unveränderte Stämme des HIV.

Bisherige Replikationsassays, die in vitro auf Basis der Vermehrung von unverändertem HIV eingesetzt werden, sind Zeit- und Ressourcen - intensiv und nicht automatisierbar wie es für die Untersuchung einer großen Anzahl von Prüfsubstanzen erforderlich wäre, sie sind also in der bisher bekannten Form nicht für die Testung großer Substanzbibliotheken geeignet.

Dies ist vor allem aus dem Verhalten von vitalem HIV in vitro begründet. Das HIV induziert auf verschiedenen Zielzellen, bevorzugt auf Zellen des Immunsystems, wie z.B. H-9 T-Lymphozyten, einen charakteristischen zytopathischen Effekt, der sich lichtmikroskopisch als Synzytienbildung, also der virusinduzierten Bildung von Großzellverbänden, darstellt. Diese Synzytienbildung ist in bisherigen Verfahren der zumeist gebräuchliche Endpunkt für die Beurteilung des zytopathischen Effekts des HIV in der Zellkultur. Sie muss nach einer bestimmten Zeit im Mikroskop beobachtet werden. Diese Art der Bestimmung der Virusvermehrung bzw. deren Hemmung ist subjektiv und zeitlich sehr aufwendig. Die lichtmikroskopische Analyse des Endpunktes ist deshalb für die Massenuntersuchung im industriellen Maßstab nicht geeignet.

Neben der direkten mikroskopischen Analyse sind als weitere Verfahren zur Auswertung HIV-spezifischer Veränderungen an Zellkulturen sogenannte Sekundärtests, wie die Messung Virus-spezifischer Proteine (z.B. in ELISA Verfahren) oder Enzymaktivitäten (z.B. HIV-Reverse Transkriptase) oder von viraler Nukleinsäure (z.B. PCR) aus dem Überstand von Zellkulturen, bekannt.

All diese Verfahren sind jedoch außerordentlich ressourcenintensiv und daher für die Testung großer Substanzbanken nicht geeignet.

Es stellt sich daher die Aufgabe, ein für das High-Throughput-Screening (HTS) geeignetes Replikationsassay zu etablieren, das unter Verwendung von natürlichem HIV und suspendierten Wirtszellen, wie beispielsweise in permanenten Zellinien gehaltenen humanen T-Lymphozyten, als Primärtest unter Verwendung eines optischen Read-outs bei gutem Signal/Rauschverhältnis durchgeführt werden kann.

Unter geeigneten Replikationsassays sind erfindungsgemäß solche Assays zu verstehen, die unter Einsatz von Suspensionszellen als Ein-Topf Assay ohne weitere Aufarbeitungsschritte durchgeführt werden können.

Im Rahmen der Erfindung wurde jetzt überraschenderweise gefunden, dass HIV und insbesondere das LAV-Isolat von HIV sich auch in Anwesenheit wesentlich geringerer als im Stand der Technik bisher gebräuchlicher Serumkonzentrationen vermehrt. Bisher gängige Wachstumsbedingungen für Wirtszellen des HIV waren Wachstumsmedien, die mindestens 20 % foetales Kälberserum (FCS) enthalten.

Dies ist deshalb von besonderer Bedeutung, da hohe Serumkonzentrationen in Zellkulturmedien von viralen Replikationssystemen an Suspensionszellen für optische Detektionsverfahren auf Basis von Fluoreszenz oder Absorptionsmessverfahren ungeeignet sind, was bedingt wird durch das Vorkommen von störenden Proteinen im FCS und der von ihnen ausgehenden enzymatischen Aktivität. Die Anwendung optischer Detektionsverfahren blieb daher bisher auf Zellkultursysteme mit adhärierenden Zellen beschränkt, bei denen sich die im Überstand der Kultur befindlichen, den optischen Test störende Proteine, leicht abpipettieren lassen.

Die vorliegenden Untersuchungen haben nun gezeigt, dass auch deutlich geringere Serumkonzentrationen ausreichen, um das HIV und insbesondere das LAV-Isolat von HIV, zu propagieren. Es wurde jetzt nämlich überraschend gefunden, dass HIV sich auch bei bisher nicht etablierten geringen FCS Serumkonzentrationen wie zum Beispiel 0,2 bis 2 %, in Suspensionszellen vermehrt, einer Konzentration, bei der die im FCS enthaltenen Störproteine mit einigen optischen Detektionsverfahren nicht länger interferieren.

Basierend auf dieser Beobachtung wird mit der jetzt vorliegenden Erfindung ein (HTS) Screening Verfahren zur schnellen, kostengünstigen und infektionsrelevanten Prüfung von großen Substanzbanken mit mehr als 200 000 Testverbindungen, bevorzugt mit mehr als 500 000 Testverbindungen oder noch größeren Substanzbibliotheken mit mehr als 1 Mio. Prüfverbindungen mit dem Ziel der Auffindung neuer Hemmstoffe des HIV vorgestellt, das darauf beruht, dass der Literatur-bekannte Replikationstest zur in vitro Vermehrung des HIV, der bisher nur in kleinem Maßstab auch zur Prüfung von Wirkverbindungen gegen das HIV einsetzbar war, nach gezielten Veränderungen in den Testbedingungen nun auch als HTS durchführbar ist.

Im Rahmen der Erfindung wurde weiter gefunden, dass es HIV-Isolate gibt, die unter den erfindungsgemäß niedrigen Serumkonzentrationen nach Infektion die Wirtszellen derartig stören und beeinflussen, dass Stoffwechselprozesse verändert werden, die dann zur Detektion von Virus-zerstörenden Prozessen verwendet werden können.

Ein in Verbindung mit niedrigen FCS-Konzentrationen erfindungsgemäß zu verwendendes HIV-Isolat ist beispielsweise das LAV Isolat des HIV, das H9 T-Lymphozytcnzellinien derart verändert, dass Virusinfektionen über veränderte biochemische Prozesse im optischen Test mittels Farbreagenzien sichtbar gemacht werden können, da die Infektion von H9 Zellen mit dem LAV-Isolat des HIV in Anwesenheit von geringen FCS-Konzentrationen dazu führt, dass die infizierten Zellen in einen physiologischen Zustand versetzt werden, der die Diskriminierung geschützter von viruszerstörten Zellen unter Verwendung von Farbreagentien ermöglicht.

Als für unter diesen Bedingungen zum Nachweis geeignete Farbreagentien wurden überraschenderweise XTT, MTT, Neutralrot und insbesondere alamar-Blue(alamar-Blue^{™}, Laboserv GmbH, Giessen, Germany; USP 5501959) oder Fluoreszenzfarbstoffe wie z.B. Fluoresceindiazetat aufgefunden.

Voraussetzung für diese Art des Nachweises ist der überraschende Befund, dass sich einige infektionsrelevante Stämme von HIV, wie beispielsweise das LAV-Isolat, auch in Zellen vermehren, in deren Kulturmedium nur geringe Mengen an foetalem Kälberserum (FCS) als essentieller Wachstumsfaktor enthalten ist. Dieser Befund ist wesentliche Voraussetzung für die erfindungsgemäßen Verfahren, da die bisher für essentiell gehaltenen höheren Konzentrationen an FCS die Verwendung von Farbreagenzien stören und deshalb ausschließen.

Die Auffindung der durch das LAV-Isolat induzierten zytopathischen und zytotoxischen Effekte unter den erfindungsgemäßen FCS-Konzentrationen in Verbindung mit den erfindungsgemäßen Farbreagenzien ermöglicht nun, ein HTS zur Auffindung von Wirkstoffen gegen das HIV als Replikationsassay durchzuführen.

Weiterhin wurde überraschenderweise gefunden, dass einige HIV-Isolate - insbesondere z.B. das LAV-Isolat - die Wirtszellen in vitro spezifisch schädigen, so dass die anschließende Detektion der Schädigung mit Hilfe von Farbstoffen möglich wird, die sonst bei den beschriebenen Verfahren nicht verwendbar sind.

Es wurde weiter gefunden, dass als Farbstoffe erfindungsgemäß alamar-Blue und Fluoreszenzfarbstoffe wie z.B. Fluoresceindiazetat eingesetzt werden können, die mit Hilfe optischer Verfahren unter Einsatz der HTS-Technik an Suspensionszellen gemessen werden können.

Es wurde weiter gefunden, dass die erfindungsgemäße Kombination bestehend aus natürlichem HIV-Isolat, Zellkulturmedium mit erfindungsgemäß niedriger FCS-Konzentration, erfindungsgemäßem Detektionsfarbstoff und suspendierten H9 T-Lymphozyten in einem HTS als Ein-Schritt-Verfahren so konfigurierbar ist, das dieses in 384-well oder auch 1536-well Platten durchgeführt werden kann.

Es wurde weiter gefunden, dass die erfindungsgemäße Kombination bestehend aus natürlichem HIV-Isolat, Zellkulturmedium mit erfindungsgemäß niedriger FCS-Konzentration, erfindungsgemäßem Detektionsfarbstoff und suspendierten H9 T-Lymphozyten in einem HTS als Ein-Schritt-Verfahren so konfigurierbar ist, dass dieses zur schnellen, und kostengünstigen Auffindung von neuen Wirkstoffen gegen HIV verwendet werden kann.

Neue Wirkstoffe im Sinne der Erfindung sind bevorzugt kleinmolekulare Wirkstoffe, die in die Replikation des HI-Virus eingreifen und dessen Vermehrung spezifisch hemmen, wobei unter kleinmolekular im Rahmen der Erfindung Verbindungen mit einem Molekulargewicht im Bereich von etwa 100 bis 500 oder 100 bis 1000 und darüber hinaus verstanden werden.

Als potentielle Hemmstoffe können aber auch hochmolekulare Verbindungen, wie z. B. Antikörper, die in großer Anzahl in Bibliotheken hergestellt werden können, in Frage kommen.

Weiterhin eignet sich der Test, große Mengen an Testproben zu überprüfen, die im klinischen Alltag von Patienten anfallen, wie z.B. Serum- oder Plasmaproben von Patienten, deren Virustiter festgestellt werden muss, um in der Verlaufskontrolle die Viruslast zu bestimmen und festzustellen, wann eine Therapie erforderlich ist. Ebenfalls fallen im klinischen Alltag eine Vielzahl von Testproben an, die von Patienten stammen, die auf Resistenzentwicklung hin überprüft werden müssen.

Bevorzugt ist das erfindungsgemäße Testverfahren jedoch zur Testung großer Substanzbibliotheken und damit zur Auffindung neuer Stoffklassen mit Wirkung gegen das HIV einsetzbar.

Unter den erfindungsgemäßen Testverfahren werden insbesondere solche Testverfahren verstanden, in denen gegenüber nativen Viren, insbesondere HI-Viren empfindliche Zielzellen, insbesondere Lymphozyten wie H9 T-Lymphozyten in Suspension in einem Zellkulturmedium, beispielsweise RPMI-Medium, das nur geringe Mengen eines Wachstumsfaktors, beispielsweise foetales Kälberserum, enthält, und der in einer Konzentration von 0,1 bis 5 Vol % , vorzugsweise 1 bis 2 Vol % vorliegt, in multi-well Platten in einem Temperaturbereich von 20 bis 40°C, vorzugsweise 30 bis 40°C und ganz bevorzugt 35 bis 38°C und bei einer CO₂- Konzentration von 2 bis 8 %, vorzugsweise 5 %, zusammen mit besagten Vieren und einer Test- bzw. Referenzsubstanz für einen Zeitraum von 2-20 Tagen, vorzugsweise 5 bis 10 Tagen, bei der für die jeweilige Temperatur sich ergebenden Sättigungsfeuchte kultiviert werden, anschließend als Read-out ein Färbemittel, vorzugsweise alamar-Blue oder Fluoresceindiazetat zugegeben, dann erneut inkubiert und nachfolgend im HTS Verfahren optisch vermessen werden.

Im Einzelnen wird dabei beispielhaft so vorgegangen, dass jeweils 3x10³ mit HIV infizierte H9-Zellen pro well einer 384-well Mikrotiterplatte eingesät werden, die geeignete Verdünnungen der Testsubstanzen bzw. Referenzsubstanz enthalten.

Unter geeigneter Verdünnung im Sinne der Erfindung werden Testsubstanzkonzentrationen zwischen 0,1 bis 50 uM verwendet, bevorzugt werden Testsubstanzkonzentrationen von 1 bis 20 uM verwendet.

Die Testplattcn werden nachfolgend über 7 Tage bei 37°C unter 5 % CO₂ und Sättigungsfeuchte im Brutschrank inkubiert. Anschließend wird zur Bestimmung der antiviralen Effekte bzw. der Zellverträglichkeit der Testsubstanzen als Detektionsfarbstoff Alarmar Blue (Biosource International) (oder Fluoresceindiazetat (200 ug/ml in PBS) in einer Menge von 1/10 Volumenteil des Weil-Inhaltes hinzugegeben. Anschließend wird im Falle von alamar-Blue für weitere 5 bis 7 Stunden bis zu 24 Stunden bei o.g. Bedingungen inkubiert. Dann erfolgt die photometrische Auswertung durch Messung von Absorption bzw. Emission. Im Falle von Fluoresceindiazetat wird die Fluoreszenz-Emission bereits nach 20 bis 60 Min. Inkubation gemessen.

Unter diesen Testbedingungen wurde für die Zellkontrolle eine optische Dichte von 1,9 Extinktionseinheiten gemessen , während die Viruskontrolle eine optische Dichte von 0,02 Extinktionseinheiten aufwies. Dieses erstaunlich günstige Signal/Rauschverhältnis von etwa 1:100 ist eine optimale Voraussetzung zur Durchführung eines HTS Assays mit HIV im Sinne der gestellten Aufgabe.

Demgegenüber findet man bei Verwendung der in dieser Technologie bisher gebräuchlichen hohen FCS-Konzentrationen in der HIV infizierten Zellkultur (20 Vol.% FCS) Extinktionswerte für die Zellkontrolle in Höhe von 1,5 Extinktionseinheiten und in der Viruskontrolle 0,4 Extinktionseinheiten.

Dieses geringe Signal/Rauschverhältnis von etwa 1:4 ist nicht ausreichend für die Durchführung eines sensitiven HTS.

## Patentansprüche

1. Replikationsassay zu Auffinden antiviral wirksamer Hemmstoffe, bestehend aus der Kombination von einem natürlichem Virus-Isolat, suspendierten Zielzellen und Zellkulturmedium enthaltend foetales Kälberserum **dadurch gekennzeichnet, dass** die Konzentration des foetalen Kälberserum weniger als 5 Vol.% beträgt.

2. Assay nach Anspruch 1 zum Auffinden von Hemmstoffen des HIV wobei das Virus-Isolat ein Isolat des HIV ist.

3. Assay nach Anspruch 1 oder 2 zum Auffinden von Hemmstoffen des HIV wobei das Virus-Isolat ein Isolat des natürlichen HIV ist.

4. Assay nach den Ansprüchen 1 bis 3 zum Auffinden von Hemmstoffen des HIV wobei das Virus-Isolat das LAV-Isolat von HIV ist.

5. Assay nach einem oder mehreren der Ansprüche 1 bis 4 **dadurch gekennzeichnet, dass** die Konzentration des foetalen Kälberserum weniger als 2 Vol.% beträgt.

6. Assay nach einem oder mehreren der Ansprüche 1 bis 5 **dadurch gekennzeichnet, dass** er einen oder mehrere Detektionsfarbstoffe enthält.

7. Assay nach einem oder mehreren der Ansprüche 1 bis 6 **dadurch gekennzeichnet, dass** er als Detektionsfarbstoffe alamar-Blue oder Fluoresceindiazetat enthält.

8. Assay nach einem oder mehreren der Ansprüche 1 bis 7 **dadurch gekennzeichnet, dass** er als Zielzellen H-9 T-Lymphozyten enthält.

9. Verwendung des Assay nach einem oder mehreren der Ansprüche 1 bis 7 im HTS.

## Claims

1. Replication assay for finding antivirally effective inhibitors, consisting of the combination of a natural virus isolate, suspended target cells and cell culture medium containing fetal calf serum, **characterized in that** the concentration of the fetal calf serum is less than 5 vol.-%.

2. Assay of claim 1 for finding inhibitors of HIV, whereby the virus isolate is an isolate of HIV.

3. Assay of claim 1 or 2 for finding inhibitors of HIV, whereby the virus isolate is an isolate of natural HIV.

4. Assay of claims 1 to 3 for finding inhibitors of HIV, whereby the virus isolate is the LAV isolate of HIV.

5. Assay of one or several of claims 1 to 4, **characterized in that** the concentration of the fetal calf serum is less than 2 vol.-%.

6. Assay of one or several of claims 1 to 5, **characterized in that** it contains one or multiple detection colourants.

7. Assay of one or several of claims 1 to 6, **characterized in that** it contains alamar blue or flurescein diacetate as detection colourants.

8. Assay of one or several of claims 1 to 7, **characterized in that** it contains H-9 T lymphocytes as target cells.

9. Use of the assay of any or several of claims 1 to 7 in a HTS.

## Revendications

1. Test de réplication pour la recherche de substances inhibitrices à activité antivirale, constitué de la combinaison d'un isolat viral naturel, de cellules cibles en suspension et d'un milieu de culture cellulaire contenant du sérum de veau foetal, **caractérisé en ce que** la concentration du sérum de veau foetal s'élève à moins de 5 % en volume.

2. Test suivant la revendication 1 pour la recherche de substances inhibitrices du virus VIH, dans lequel l'isolat viral est un isolat du virus VIH.

3. Test suivant la revendication 1 ou 2 pour la recherche de substances inhibitrices du virus VIH, dans lequel l'isolat viral est un isolat du virus VIH naturel.

4. Test suivant les revendications 1 à 3 pour la recherche de substances inhibitrices du virus VIH, dans lequel l'isolat viral est l'isolat LAV du virus VIH.

5. Test suivant l'une ou plusieurs des revendications 1 à 4, **caractérisé en ce que** la concentration du sérum de veau foetal s'élève à moins de 2 % en volume.

6. Test suivant l'une ou plusieurs des revendications 1 à 5, **caractérisé en ce qu'**il contient un ou plusieurs colorants de détection.

7. Test suivant l'une ou plusieurs des revendications 1 à 6, **caractérisé en ce qu'**il contient du Bleu Alamar ou du diacétate de fluorescéine comme colorants de détection.

8. Test suivant l'une ou plusieurs des revendications 1 à 7, **caractérisé en ce qu'**il contient des lymphocytes T H-9 comme cellules cibles.

9. Utilisation du test suivant l'une ou plusieurs des revendications 1 à 7 dans la méthode HTS.
